# EUROPÄISCHE PATENTSCHRIFT

(11) **EP 1 797 024 B1**
(45) Veröffentlichungstag und Bekanntmachung des Hinweises auf die Patenterteilung: **19.06.2013**
(21) Anmeldenummer: 05784465.6
(22) Anmeldetag: 15.09.2005
(51) Int. Cl.: C07C 2/66, C07C 15/073, C07C 7/13

(54) **VERFAHREN ZUR REINIGUNG VON AROMATEN ENTHALTENDEN ZUFUHRSTRÖMEN MIT ZEOLITHEN**
METHOD FOR PURIFYING SUPPLY FLOWS CONTAINING AROMATICS BY MEANS OF ZEOLITES
PROCEDE DE PURIFICATION DE FLUX D'ALIMENTATION CONTENANT DES COMPOSES AROMATIQUES AU MOYEN DE ZEOLITHES

(30) Priorität: 20.09.2004 DE 102004045879
(43) Veröffentlichungstag der Anmeldung: 20.06.2007
(73) Patentinhaber: Styrolution GmbH, 60325 Frankfurt am Main (DE)
(72) Erfinder: HENN, Rolf, 68723 Oftersheim (DE); MÜLLER, Ulrich, 67435 Neustadt (DE); STRAUB, Ferdinand, 68766 Hockenheim (DE); DOSCH, Jürgen, 67063 Ludwigshafen (DE)
(74) Vertreter: Jacobi, Markus Alexander
(86) Internationale Anmeldenummer: PCT/EP2005/009909
(87) Internationale Veröffentlichungsnummer: WO 2006/032400

(56) Entgegenhaltungen:
- WO-A-98/07673
- US-A- 5 942 650

## Beschreibung

Die Erfindung betrifft ein Verfahren zur Reinigung von Aromaten enthaltenden Zufuhrströmen in Polymerisations- oder Alkylierungsverfahren durch Inkontaktbringen mit Zeolithen, dadurch gekennzeichnet, dass man den Zufuhrstrom über mindestens zwei Zeolithe 1 und 2 leitet, wobei der Zeolith 1 eine mittlere Porengröße von 0,3 bis 0,5 nm, und der Zeolith 2 mit eine mittlere Porengröße von 0,6 bis 0,8 nm, aufweist.

Alkylierte Aromaten werden überwiegend durch katalytische Alkylierung von Aromaten mit Olefinen erhalten, Ethylbenzol beispielsweise durch Alkylierung von Benzol mit Ethylen, Cumol beispielsweise durch Alkylierung von Benzol mit Propylen. Als Katalysatoren werden in flüssiger Phase Aluminiumchlorid und in der Gasphase LewisSäuren oder synthetische Zeolithe verwendet. Zeolithe sind hochaktive Katalysatoren sowohl für die Alkylierung als auch für die Transalkylierung. Da die Zeolithkatalysatoren anfällig auf Wasser, Schwefel und andere Katalysatorgifte reagieren, verlieren sie mit der Zeit Ihre Aktivität und müssen periodisch regeneriert werden.

Zur Verlängerung der Lebensdauer von Zeolithkatalysatoren für Alkylierungsreaktionen wurden verschiedene Verfahren vorgeschlagen. Die WO 98/07673 beschreibt die Alkylierung von Benzol mit beispielsweise Propylen. Das Benzol wird durch Überleitung über Aluminiumoxide, Silikate, Aluminiumsilikate oder saure Zeolithe wie z.B. Mordenite vorbehandelt.

In der WO 00/35836 wird beschrieben, zunächst einen Aromaten zu alkylieren, den erhaltenen Alkylaromaten mit einem Molekularsieb zu reinigen und schließlich den gereinigten Alkylaromaten mit einem anderen Aroamten in einer Transalkylierungsreaktion zu einem Monoalkylaromaten umzusetzen. Als Molekularsiebe sind bestimmte Zeolithe und deren Mischungen geeignet, wobei saure Zeolithe wie MCM-22 bevorzugt sind. Inzwischen wurde jedoch gefunden, dass die Wirksamkeit dieser sauren Zeolithe unzureichend ist.

Aus der US-A-5,942,650 ist ein Verfahren bekannt, bei dem der Aromaten enthaltende Zufuhrstrom nur über einen Zeolithen mit einer mittleren Porengröße von weniger als 0,5nm geleitet wird.

Die WO 01/07383 offenbart die Reinigung von Olefine enthaltenden Zufuhrströmen in Polymerisations- oder Alkylierungsverfahren durch Überleiten des Olefins, z.B. Ethylen, über eine Adsorptionsschicht aus Ruß, Aktivkohle, Aluminiumoxiden, Silikaten, Aluminiumsilikaten, verschiedensten Zeolithen oder Molekularsieben. Es wird erwähnt, dass zweckmäßigerweise auch der Benzol- bzw. Alkylbenzol-Zufuhrstrom über eine entsprechende Adsorptionsschicht aus den genannten Adsorptionsmitteln geleitet wird; nähere Angaben werden nicht gemacht.

Die Reinigungswirkung dieser Verfahren für Aromaten ist nicht in allen Fällen zufriedenstellend, oder es sind teure Adsorbentien erforderlich.

Es bestand die Aufgabe, ein verbessertes Verfahren zur Reinigung von Aromaten bereitzustellen, insbesondere von solchen Aromaten, die in einem Polymerisations- oder Alkylierungsverfahren eingesetzt werden sollen. Dieses Reinigungsverfahren sollte die Lebensdauer von Alkylierungs- bzw. Transalkylierungskatalysatoren bei der katalytischen Alkylierung von Aromaten mit Olefinen, insbesondere an Zeolithkatalysatoren, verlängern, und den zur Regenerierung erforderlichen Aufwand zu verringern.

Demgemäß wurde das eingangs definierte Verfahren (nachfolgend auch als Reinigungsverfahren bezeichnet) gefunden. Außerdem wurde ein Verfahren (Alkyllerungsverfahren) zur Herstellung von alkylierten Aromaten durch Umsetzung von Aromaten und Olefinen an einem Katalysator gefunden, dadurch gekennzeichnet, dass man den Aromaten enthaltenden Zufuhrstrom mit dem Reinigungsverfahren vorbehandelt. Bevorzugte Ausführungsformen der Erfindung sind den Unteransprüchen zu entnehmen.

Prinzipiell kann das erfindungsgemäße Reinigungsverfahren auch für Aromaten enthaltende Zufuhrströme in anderen Verfahren verwendet werden. Besonders geeignet ist es jedoch für Polymerisations- und Alkylierungsverfahren, insbesondere solche, bei denen Katalysatoren zum Einsatz kommen, die auf geringste Mengen an Verunreinigungen empfindlich sind.

Als zu reinigende Aromaten eignen sich sowohl nicht alkylierte Aromaten, die z.B. mit einem Olefin in einer Alkylierungsreaktion zu alkylierten Aromaten umgesetzt werden können, als auch ein- oder mehrfach alkylierte Aromaten, die z.B. mit anderen Aromaten in einer Transalkylierungsreaktion zu anderen alkylierten Aromaten umgesetzt wer den können. Als nicht alkylierte Aromaten kommen beispielsweise Benzol und kondensierte Aromaten wie Naphthalin oder Anthracen in Betracht. Als alkylierte Aromaten eignen sich solche mit 1 bis 10 C-Atomen im Alkylrest, beispielsweise monoalkylierte Aromaten wie Toluol oder Ethylbenzol oder polyalkylierte Aromaten wie die Xylole. Bevorzugt verwendet man als zu reinigenden Aromat Benzol.

Zweckmäßigerweise wird der Aromat bis auf einen Wassergehalt unter 100, bevorzugt unter 30 ppm by weight (gemessen nach Karl Fischer gemäß DIN 51777) entwässert, bevor er dem Reinigungsverfahren zugeführt wird. Dies erfolgt in üblicher Weise, bei kontinuierlicher Durchführung des Verfahrens z.B. durch Trocknungskolonnen.

Erfindungsgemäß wird der Aromaten enthaltende Zufuhrstrom mit Zeolithen in Kontakt gebracht, indem man den Zufuhrstrom über mindestens zwei Zeolithe 1 und 2 leitet. Die Bezeichnungen Zeolith 1 bzw. Zeolith 2 dienen lediglich der sprachlichen Unter scheidung der beiden Zeolithe voneinander im Sinne einer besseren Textverständlichkeit und bezeichnen keinen bestimmten Zeolith-Strukturtyp.

Der Zeolith 1 hat eine mittlere Porengröße von 0,3 bis 0,5 nm (3 bis 5 Å), und der Zeolith 2 hat eine mittlere Porengröße von 0,6 bis 0,8 nm (6 bis 8 Å). Demnach ist Zeolith 1 kleinporig und Zeolith 2 mittelporig bis großporig.

In einer bevorzugten Ausführungsform des Verfahrens verwendet man, bezogen auf die Summe aus Zeolith 1 und Zeolith 2,
a) 10 bis 90, vorzugsweise 30 bis 70 und insbesondere 40 bis 60 Gew.-% des Zeoliths 1, und
b) 10 bis 90, vorzugsweise 30 bis 70 und insbesondere 40 bis 60 Gew.-% des Zeoliths 2.

In einer ebenfalls bevorzugten Ausführungsform weist der Zeolith 1 eine mittlere Porengröße von 0,38 bis 0,42 nm (3,8 bis 4,2 Å), insbesondere etwa 0,4 nm (4 Å), und der Zeolith 2 eine mittlere Porengröße von 0,68 bis 0,72 nm (6,8 bis 7,2 Å), insbesondere etwa 0,7 nm (7 Å), auf.

Als Zeolith 1 eignen sich beispielsweise Zeolithe des Strukturtyps LTA mit Porengrößen von 0,3 bis 0,5 nm. Besonders bevorzugte Zeolithe 1 mit Porengrößen von etwa 0,4 nm sind LTA-Zeolithe in der Natriumform.

Als Zeolith 2 eignen sich beispielsweise Zeolithe des Strukturtyps FAU mit Porengrö-βen von 0,6 bis 0,8 nm. Besonders bevorzugte Zeolithe 2 mit Porengrößen von etwa 0,7 nm sind FAU-Zeolithe in der Natriumform oder Calciumform.

Bevorzugt sind die verwendeten Zeolithe 1 und 2 keine sauren bzw. sauer aktivierten Zeolithe. Besonders bevorzugt verwendet man neutrale Zeolithe 1 bzw. 2.

Es versteht sich, dass als Zeolith 1 auch Mischungen mehrerer Zeolithe 1', 1", etc., und als Zeolith 2 auch Mischungen verschiedener Zeolithe 2', 2", etc., verwendet werden können.

Die genannten Zeolithe sind bekannt und handelsüblich. Struktur, Eigenschaften und Herstellung von Zeolithen sind beispielsweise in Zeolite Molecular Sieves, Donald W. Breck, John Wiley&Sons, 1974; in Atlas of Zeolite Framework Types, Ch. Baerlocher/W.M. Meier/D.H. Olson, 5th Ed., Elsevier 2001; oder in Handbook of Molecular Sieves, R. Szostak, Chapman&Hall, New York, 1992 beschrieben.

Im allgemeinen werden die Zeolithe In Form von Kugeln, Stäbchen oder Granulaten mit einer Außenabmessung von 0,5 bis 10 mm eingesetzt.

Bei dem erfindungsgemäßen Verfahren können die Zeolithe als festes, bewegtes oder fluidisiertes Bett vorliegen. Bevorzugt liegen die Zeolithe als Festbett vor. Man kann die Zeolithe 1 und 2 mischen und diese Mischung als festes, bewegtes oder fluidisiertes Bett verwenden, oder - und dies ist bevorzugt - man kann den Zeolith 1 und den Zeolith 2 getrennt voneinander in verschiedenen Betten anordnen, wobei die Betten hinter einander (seriell) angeordnet sind und unabhängig voneinander fest, bewegt oder fluidisiert sein können. Bevorzugt liegen beide Zeolithe 1 und 2 als Festbett vor.

Es versteht sich, dass jedes Bett als mehrere aufeinanderfolgende Betten ausgeführt sein kann.

Das erfindungsgemäße Verfahren kann absatzweise oder kontinuierlich durchgeführt werden. Die apparative Ausgestaltung des oder der Zeolithbetten ist die übliche; beispielsweise kann man das Zeolithbett in einem Absorber oder einem anderen geeigneten Behälter anordnen, der vom zu reinigenden Aromaten durchströmt wird. Bevorzugt verwendet man Festbettabsorber. Der Absorber bzw. sonstige Behälter ist bevorzugt zu 70 bis 90 Vol.% seines Volumens mit den Zeolithen gefüllt.

Nach einer gewissen Standzeit sind die Zeolithe mit Verunreinigungen beladen und die Reinigungsleistung lässt nach. Die Regeneration (Entfernung der adsorbierten Verunreinigungen) erfolgt in üblicher Weise, z.B. durch mehrstündiges Behandeln des Zeolithbettes mit heißen Intertgasen bei 200 bis 400°C. Man kann zwei oder mehrere Zeolithbetten nebeneinander (parallel) anordnen und über das Bett den zu reinigenden Aromaten leiten, während das andere Bett mit heißem Innertgas regeneriert wird.

Die Größe des Adsorbers, die Art und Menge der Zeolithe und die Strömungsgeschwindigkeit des Zufuhrstromes bzw. die Verweilzeit im Absorber hängen von der Art und Menge der Verunreinigungen, der erforderlichen Reinigungsleistung (tolerierbare Konzentration der Verunreinigungen im gereinigten Aromaten) und den gewünschten Regenerierungszyklen ab.

Sofern man die Zeolithe 1 und 2 nicht als homogene Mischung, sondern getrennt voneinander verwendet, ist es bevorzugt, den zu reinigenden Aromaten in Strömungsrichtung betrachtet zunächst über den großporigen Zeolith 2 und danach über den kleinporigen Zeolith 1 zu leiten, d.h. das Bett aus Zeolith 1 hinter dem Bett aus Zeolith 2 anzuordnen. Jedoch kann in bestimmten Fällen auch die umgekehrte Reihenfolge vorteilhaft sein.

Die Anordnung Zeolith 2 vor Zeolith 1 kann bei einem Festbett in einfacher Weise dadurch erfolgen, dass man in den Adsorber zunächst eine Schicht aus großporigem Zeolith 2 einfüllt und auf diese erste Schicht eine zweite Schicht aus kleinporigem Zeolith 1 gibt. Der Verunreinigungen enthaltende Zufuhrstrom wird dann am Boden des ordnen. Jedoch kann in bestimmten Fällen auch die umgekehrte Reihenfolge vorteilhaft sein.

Die Anordnung Zeolith 2 vor Zeolith 1 kann bei einem Festbett in einfacher Weise dadurch erfolgen, dass man in den Adsorber zunächst eine Schicht aus großporigem Zeolith 2 einfüllt und auf diese erste Schicht eine zweite Schicht aus kleinporigem Zeolith 1 gibt. Der Verunreinigungen enthaltende Zufuhrstrom wird dann am Boden des Adsorbers zugeführt und am Kopf gereinigt abgezogen. Für die umgekehrte Anordnung Zeolith 1 vor Zeolith 2 ist naturgemäß zunächst eine Schicht aus Zeolith 1 und darauf eine Schicht aus Zeolith 2 einzufüllen.

Bevorzugt leitet man den Zufuhrstrom bei einer Temperatur von 0 bis 300°C, insbesondere 50 bis 200°C und besonders bevorzugt 100 bis 150°C und einem Druck von 1 bis 50 × 10⁵ Pa (1-50 bar), insbesondere 3 bis 30 × 10⁵ Pa (3-30 bar) und besonders bevorzugt 5 bis 20 x 10⁵ Pa (5-20 bar) über die Zeolithe. Dabei kann man für Zeolith 1 und Zeolith 2 bzw. für die verschiedenen Zeolithbetten gleiche oder verscheiden Temperaturen bzw. Drucke einstellen, je nach Art und Menge der Verunreinigungen und der erforderlichen Reinigungsleistung.

Der zu reinigende Aromat wird beispielsweise durch Destillation aus Aromatengemischen oder einer als Hydrodealkylierung bekannten katalytischen Umwandlung von Aromatengemischen gewonnnen. Typische Verunreinigungen des Zufuhrstromes sind daher solche, die in der Extraktivdestillation von Aromatengemischen anfallen, insbesondere N-Methylpyrrolidon, N-Formylmorpholin und Sulfolan. Derartige stickstoff- oder schwefelhaltige Verunreinigungen können beispielsweise durch Chemolumineszenz oder andere dem Fachmann als geeignet bekannte analytische Verfahren bestimmt werden. Typischerweise beträgt der Stickstoffgehalt im Zufuhrstrom, gerechnet als N₂, etwa 0,1 bis 10, insbesondere 0,5 bis 5 ppm by weight, z.B. etwa 1 ppm by weight pro einzelner Verunreinigung und bezogen auf den nicht gereinigten Aromaten, z.B. Benzol. Bereits bei Konzentrationen von 0,5 bis 1 ppm N₂ wird die Leistung des Katalysators beeinträchtigt.

Die Reinigungsleistung und damit die Güte des erfindungsgemäßen Reinigungsverfahrens lässt sich am einfachsten am Verhalten der Katalysatoren beurteilen, die bei den Polymerisations- oder Alkylierungsverfahren verwendet werden, in denen der erfindungsgemäß gereinigte Aromat weiterverwendet wird. Je länger die Lebensdauer (Standzeit) dieser Polymerisations- bzw. Alkylierungskatalysatoren ist, desto geringer ist die Konzentration der Verunreinigungen in den Einsatzstoffen, und umso besser ist die Reinigungsleistung des Reinigungsverfahrens, mit dem die Einsatzstoffe zuvor gereinigt wurden.

Insbesondere die bei Alkylierungsreaktionen verwendeten Katalysatoren binden Verunreinigungen stark und erschöpfen bei unzureichender Reinigung der Einsatzstoffe lytisch wirksam ist. Wenn die reaktive Zone schließlich am Ende (Austritt) des Festbettes angekommen ist, ist die gesamte Katalysatormenge desaktiviert.

Messbar wird dieser Effekt z.B. durch Temperaturmessungen im Katalysatorfestbett: entlang des Festbettes in Strömungsrichtung hintereinander liegende Temperatur messpunkte zeigen das Profil der exothermen Reaktion über das Festbett an. Steigt die Temperatur am Festbettanfang stark an, bezogen auf die Temperatur des Zufuhr stroms, erfolgt hier ein wesentlicher Teil des Umsatzes. Ist der Temperaturanstieg am Festbettanfang gering aber stromabwärts hoch, hat sich der Umsatz stromabwärts verlagert. (Erhöht sich auch am Festbettende die Temperatur nicht, ist das Katalysatorbett auf ganzer Länge erschöpft und muss ausgetauscht oder regeneriert werden.)

Das erfindungsgemäße Reinigungsverfahren ist ökonomischer als die Verfahren des Standes der Technik. Insbesondere weisen die Polymeriations- bzw. Alkylierungskatalysatoren eine längere Lebensdauer auf, wenn ein erfindungsgemäß gereinigter Aromat verwendet wird. Dies vermindert den Aufwand für die Katalysatorregenerierung erheblich.

Ein weiterer Gegenstand der Erfindung ist ein Verfahren zur Herstellung von alkylierten Aromaten (Alkylierungsverfahren) durch Umsetzung von Aromaten und Olefinen an einem Katalysator, dadurch gekennzeichnet, dass man den Aromaten enthaltenden Zufuhrstrom mit dem erfindungsgemäßen Verfahren gemäß den Ansprüchen 1 bis 6 (Reinigungsverfahren) vorbehandelt Als Katalysator eignen sich insbesondere LewisSäuren oder Zeolithe. Alkylierung schließt die Transalkylierung mit ein.

Solche Alkylierungsverfahren sind z. B. in Ullmann, Encycl. of Industrial Chemistry, 5th Ed. Vol A10, Seiten 35 bis 43 beschrieben. Besonders bevorzugt wird es in der Zeolithkatalysierten Alkylierung oder Transalkylierung von Benzol und Ethylen eingesetzt. Solche Verfahren und geeignete Katalysatoren sind beispielsweise in US 5,902,917, US 4,891,448, US 5,081,323, US 5,198595, US 5,243,116 oder WO 98/07673 beschrieben.

Bevorzugt verwendet man in dem erfindungsgemäßen Alkylierungsverfahren als Aromat Benzol, und als Olefin Ethylen (womit Ethylbenzol erhalten wird) oder Propylen (womit Cumol erhalten wird).

Sofern im Alkylierungsverfahren Zeolithe als Katalysatoren verwendet werden, sind diese bevorzugt verschieden von den Zeolithen, die im Reinigungsverfahren eingesetzt werden. Man kann gebrauchte Katalysatoren als "guard bed" verwenden.

Zweckmäßigerweise wird bei der Alkylierung von Aromaten, neben dem Aromat-Zufuhrstrom auch der Olefin (oder, im Falle der Transalkylierung, andere Alkylaromaten) enthaltende Zufuhrstrom gereinigt. Beispielsweise kann man bei der Ethylbenzolherstellung neben dem Benzol auch das Ethylen reinigen. Dazu kann man beispielsweise den Olefin-Zufuhrstrom über eine geeignete Adsorptionsschicht leiten, wie dies in der WO 01/07383 beschrieben ist

Das erfindungsgemäße Verfahren verbessert die Reinigung von Aromaten, insbesondere von solchen Aromaten, die in einem Polymerisations- oder Alkylierungsverfahren eingesetzt werden sollen. Das Verfahren verlängert die Lebensdauer von Alkylierungs- bzw. Transalkylierungskatalysatoren bei der katalytischen Alkylierung von Aromaten mit Olefinen, insbesondere an Zeolithkatalysatoren, und verringert den zur Regenerierung erforderlichen Aufwand.

### Beispiele

Es wurden folgende Einsatzstoffe verwendet

| | |
|---|---|
| Zeolith 1: | Zeolith Typ Z4-04 von Fa. Zeochem, Schweiz, ein Zeolith mit einer mitt leren Porengröße von 0,4 nm (4 A) in Form von Kugeln mit einem Durchmesser von 2 bis 3 mm, Schüttdichte ca. 730 kg/m³ |
| Zeolith 2: | Zeolith Typ Z10-03 von Fa. Zeochem, ein Zeolith mit einer mittleren Porengröße von 0,7 nm (7 Å) in Form von Kugeln mit einem Durchmesser von 1,6 bis 2,3 mm, Schüttdichte ca. 650 kg/m³ |
| Benzol: | Benzol wurde in einer vorgeschalteten Trocknungskolonne auf einen Wassergehalt kleiner 30 ppm by weight (gemessen nach Karl Fischer gemäß DIN 51777) azeotrop entwässert |
| Ethylen: | aus dem Steamcracker der BASF in Ludwigshafen. |

### Beispiel 1 (zum Vergleich):

In einen Adsorberturm mit 200 cm Durchmesser und 35 m³ Volumen wurden 20 t des großporigen Zeolith 2 eingefüllt und zu einem Festbett von 10 m Höhe verteilt. Man leitete kontinuierlich Benzol am Boden des Adsorberturms ein und zog das gereinigte Benzol am Kopf des Turms ab. Der Massestrom des Benzols betrug 60 bis 70 t/h, was einer Volumenströmung von 2 h⁻¹ entsprach. Die Temperatur des zugeführten Benzols betrug ca. 130°C.

Das erhaltene gereinigte Benzol wurde mit Ethylen im Masseverhältnis 55: 1 (Benzol-überschuss) gemischt und das Gemisch Ober einen Festbettreaktor geleitet, der einen Zeolith-Katalysator enthielt. Als Produkt dieser Alkylierungsreaktion erhielt man ein Gemisch aus nicht umgesetztem Benzol, Ethylbenzol und mehrfach alkylierten Benzolen.

Entlang des Festbettreaktors der Alkylierungsreaktion waren vier Temperaturmessfühler x1 bis x4 angebracht, wobei sich x1 am Anfang des Katalysator-Festbettes (Eintritt der Reaktanden) und x4 am Ende des Festbettes (Produktaustritt) befand. Die Messfühler erfassten die Temperaturerhöhung ΔT der Reaktionsmischung im Festbett, hervorgerufen durch die exotherme Umsetzung. ΔT ist bezogen auf die Temperatur des Zufuhrstroms.

Der Versuch wurde nach 4 Wochen beendet, als die Temperaturerhöhung ΔT am Katalysatorbettanfang (x1) stark abgenommen hatte und eine sinkende Temperatur am Katalysatorbettende anzeigte, dass der Umsatz nicht mehr vollständig war. Dies zeigte an, dass der Katalysator desaktiviert war.

### Beispiel 2 (erfindungsgemäß):

Beispiel 1 wurde wiederholt, jedoch wurden in den Adsorberturm zunächst 14 t des großporigen Zeolith 2 eingefüllt und zu einem Festbett von 6,9 m Höhe verteilt; auf diese Schicht wurden 7 t des kleinporigen Zeolith 1 gegeben und zu einem Festbett von 3,1 m Höhe verteilt.

Ansonsten wurde vorgegangen wie in Beispiel 1 beschrieben, wobei selbstverständlich der Festbettreaktor der Alkylierungsreaktion einen frischen Zeolith-Katalysator enthielt. Die Reaktion konnte 10 Wochen durchgeführt werden, ohne dass der Katalysator desaktiviert war.

Die Tabelle fasst die Ergebnisse zusammen.

**Tabelle: Temperaturerhöhung ΔT an den Messfühlern x1 bis x4, bezogen auf die Temperatur des Zufuhrstroms (- bedeutet keine Messung da Katalysator am Bettanfang erschöpft)**

| Woche | 0¹⁾ | 1 | 2 | 3 | 4 | 5 | 6 | 7 | 8 | 9 | 10 |
|---|---|---|---|---|---|---|---|---|---|---|---|
| ΔT (x1 = Katalysatorbettanfang) | | | | | | | | | | | |
| Bsp. 1 | 15,0 | 5 | 2,8 | 2 | 1,8 | - | - | - | - | - | - |
| Bsp. 2 | 17 | 14,1 | 12,5 | 11,2 | 10,7 | 10,3 | 10 | 9,9 | 9,8 | 9,7 | 9,6 |

| ΔT (x2) | | | | | | | | | | | |
|---|---|---|---|---|---|---|---|---|---|---|---|
| Bsp. 1 | 22,4 | 16 | 11,1 | 9,8 | 9 | - | - | - | - | - | - |
| Bsp. 2 | 23,8 | 23,3 | 23 | 22,7 | 22,6 | 22,5 | 22,5 | 22,4 | 22,4 | 22,3 | 22,3 |

| ΔT(x3) | | | | | | | | | | | |
|---|---|---|---|---|---|---|---|---|---|---|---|
| Bsp. 1 | 22,8 | 22,1 | 19,9 | 18,2 | 17,2 | 17,2 | - | - | - | - | - |
| Bsp. 2 | 24,1 | 24,1 | 24,1 | 24,1 | 24,1 | 24,1 | 24 | 24 | 24 | 24 | 24 |

| ΔT (x4 = Katalysatorbettende) | | | | | | | | | | | |
|---|---|---|---|---|---|---|---|---|---|---|---|
| Bsp. 1 | 23,2 | 23,2 | 23,2 | 23,2 | 22,9 | 22,9 | - | - | - | - | - |
| Bsp. 2 | 24,4 | 24,4 | 24,4 | 24,4 | 24,4 | 24,4 | 24,4 | 24,3 | 24,3 | 24,3 | 24,3 |

| | | | | | | | | | | | |
|---|---|---|---|---|---|---|---|---|---|---|---|
| ¹⁾ Startzeitpunkt | | | | | | | | | | | |

Die Beispiele zeigen, dass beim Vergleichsbeispiel 1 bereits nach 4 Wochen Betriebsdauer der Katalysator am Anfang des Katalysatorfestbettes (x1) erschöpft war, denn in diesem Bereich war fast keine Temperaturerhöhung ΔT mehr messbar.

Beim erfindungsgemäßen Beispiel 2 betrug nach 10 Wochen Betriebsdauer die Temperaturerhöhung am Katalysatoranfang (x1) immer noch 40 % der am Katalysatorende (x4) gemessenen Temperaturerhöhung; letztere ist die maximal mögliche Temperatur erhöhung. Bereits am Messpunkt x2 betrug ΔT auch nach 10 Wochen immer noch 90 % der maximalen Erhöhung ΔT am Messpunkt x4.

Das erfindungsgemäße Reinigungsverfahren erhöhte die Lebensdauer des Katalysators erheblich.

## Patentansprüche

1. Verfahren zur Reinigung von Aromaten enthaltenden Zufuhrströmen in Polymerisations- oder Alkylierungsverfahren durch Inkontaktbringen mit Zeolithen, **dadurch gekennzeichnet, dass** man den Zufuhrstrom über mindestens zwei Zeolithe 1 und 2 leitet, wobei der Zeolith 1 eine mittlere Porengröße von 0,3 bis 0,5 nm, und der Zeolith 2 mit eine mittlere Porengröße von 0,6 bis 0,8 nm, aufweist.

2. Verfahren nach Anspruch 1, **dadurch gekennzeichnet, dass** man als Aromat Benzol verwendet.

3. Verfahren nach den Ansprüchen 1 bis 2, **dadurch gekennzeichnet, dass** man, bezogen auf die Summe aus Zeolith 1 und Zeolith 2,
a) 30 bis 70 Gew.-% des Zeoliths 1, und
b) 30 bis 70 Gew.-% des Zeoliths 2, verwendet.

4. Verfahren nach den Ansprüchen 1 bis 3, **dadurch gekennzeichnet, dass** der Zeolith 1 eine mittlere Porengröße von 0,38 bis 0,42 nm, und der Zeolith 2 eine mittlere Porengröße von 0,68 bis 0,72 nm aufweist.

5. Verfahren nach den Ansprüchen 1 bis 4, **dadurch gekennzeichnet, dass** die Zeolithe neutrale Zeolithe sind.

6. Verfahren nach den Ansprüchen 1 bis 5, **dadurch gekennzeichnet, dass** die Zeolithe als Festbett vorliegen.

7. Verfahren nach den Ansprüchen 1 bis 6, **dadurch gekennzeichnet, dass** man den Zufuhrstrom bei einer Temperatur von 50 bis 200°C und einem Druck von 1 bis 50 x 10⁵ Pa (1-50 bar) über die Zeolithe leitet.

8. Verfahren zur Herstellung von alkylierten Aromaten durch Umsetzung von Aromaten und Olefinen an einem Katalysator, **dadurch gekennzeichnet, dass** man den Aromaten enthaltenden Zufuhrstrom mit dem Verfahren gemäß den Ansprüchen 1 bis 7 vorbehandelt.

9. Verfahren nach Anspruch 8, **dadurch gekennzeichnet, dass** man als Aromat Benzol, und als Olefin Ethylen oder Propylen verwendet.

## Claims

1. A process for purifying feed streams comprising aromatics in polymerization or alkylation processes by bringing them into contact with zeolites, which comprises passing the feed stream over at least two zeolites 1 and 2, with zeolite 1 having a mean pore size of from 0.3 to 0.5 nm and zeolite 2 having a mean pore size of from 0.6 to 0.8 nm.

2. The process according to claim 1, wherein benzene is ued as aromatic.

3. The process according to claim 1 or 2, wherein, based on the sum of zeolite 1 and zeolite 2,
a) from 30 to 70% by weight of zeolite 1 and
b) from 30 to 70% by weight of zeolite 2, are used.

4. The process according to any of claims 1 to 3, wherein zeolite 1 has a mean pore size of from 0.38 to 0.42 nm and zeolite 2 has a mean pore size of from 0.68 to 0.72 nm.

5. The process according to any of claims 1 to 4, wherein the zeolites are neutral zeolites.

6. The process according to any of claims 1 to 5, wherein the zeolites are present as a fixed bed.

7. The process according to any of claims 1 to 6, wherein the feed stream is passed over the zeolites at a temperature of from 50 to 200°C and a pressure of from 1 to 50 x 10⁵ Pa (1 to 50 bar).

8. A process for preparing alkylated aromatics by reacting aromatics and olefins over a catalyst, wherein the feed stream comprising aromatics is pretreated by the process according to any of claims 1 to 7.

9. The process according to claim 8, wherein benzene is used as aromatic and ethylene or propylene is used as olefin.

## Revendications

1. Procédé de purification de flux d'alimentation contenant des composés aromatiques dans des procédés de polymérisation ou d'alkylation impliquant l'amenée de ces flux au contact de zéolites, **caractérisé en ce qu'**on fait passer le flux d'alimentation sur au moins deux zéolites 1 et 2, la zéolite 1 étant une zéolite présentant une dimension moyenne de pores de 0,3 à 0,5 nm, et la zéolite 2 étant une zéolite présentant une dimension moyenne de pores de 0,6 à 0,8 nm.

2. Procédé selon la revendication 1, **caractérisé en ce que** le composé aromatique employé est le benzène.

3. Procédé selon les revendications 1 à 2, **caractérisé en ce que**, sur la base de la somme des zéolites 1 et 2 sont employés respectivement
a) 30 à 70% en poids de zéolite 1,
b) 30 à 70% en poids de zéolite 2.

4. Procédé selon les revendications 1 à 3, **caractérisé en ce que** la zéolite 1 présente une dimension moyenne de pores de 0,38 à 0,42 nm, et la zéolite 2 présente une dimension moyenne de pores de 0,68 à 0,72 nm.

5. Procédé selon les revendications 1 à 4, **caractérisé en ce que** les zéolites sont des zéolites neutres.

6. Procédé selon les revendications 1 à 5, **caractérisé en ce que** les zéolites sont présentes sous forme de lit fixe.

7. Procédé selon les revendications 1 à 6, **caractérisé en ce que** le flux d'alimentation est amené sur les zéolites à une température de 50 à 200°C et sous une pression de 50 à 10⁵Pa (1 à 50 bars).

8. Procédé pour la préparation de composés aromatiques alkylés par transformation de composés aromatiques et d'oléfines en présence d'un catalyseur, **caractérisé en ce que** le flux d'alimentation contenant des composés aromatiques subit un prétraitement par le procédé selon les revendications 1 à 7.

9. Procédé selon la revendication 8, **caractérisé en ce que** l'on utilise comme composé aromatique le benzène et, comme oléfine, l'éthylène ou le propylène.
